# EUROPEAN PATENT APPLICATION

(11) **EP 4 550 288 A1**
(43) Date of publication of application: **07.05.2025**
(21) Application number: 23831089.0
(22) Date of filing: 14.06.2023
(51) Int. Cl.: G08B 21/02, G08B 25/00, G08B 25/04

(54) **MONITORING SYSTEM, MONITORING DEVICE, MONITORING METHOD, AND MONITORING PROGRAM**

(30) Priority: 29.06.2022 JP 2022104858
(71) Applicant: GLORY LTD., Himeji-shi Hyogo 670-8567 (JP)
(72) Inventor: KAWANO, Mikoto, Himeji-shi, Hyogo 670-8567 (JP)
(74) Representative: SSM Sandmair
(86) International application number: PCT/JP2023/021999
(87) International publication number: WO 2024/004641

(57) **Abstract**

A monitoring system (A) includes: an alarm device (2); a state detection unit configured to detect a state of a monitored person (D); an alarm control unit capable of activating the alarm device (2) in response to a predetermined state being detected by the state detection unit; and a determination unit configured to determine whether or not a predetermined attribute person (E), who is different from the monitored person (D) and has a predetermined attribute, is present in an area (B) where the monitored person (D) is present, wherein the alarm control unit does not activate the alarm device (2) in response to (i) the predetermined state being detected by the state detection unit and (ii) the determination unit determining that the predetermined attribute person (E) is present in the area (B), and the alarm control unit activates the alarm device (2) in response to (i) the predetermined state being detected by the state detection unit and (ii) the determination unit determining that the predetermined attribute person (E) is not present in the area (B).

## Description

### Technical Field

The present disclosure relates to a monitoring system, a monitoring device, a monitoring method, and a monitoring program.

### Background Art

Patent Document 1 discloses a monitoring system for monitoring a monitored person. In this monitoring system ("monitored person monitoring system" in Patent Document 1), an alarm device ("portable terminal unit" in Patent Document 1) is activated if a monitored person present in a given area performs a predetermined action.

### Prior Art Document

### Patent Document

Patent Document 1: JP 6123971B

### Disclosure of the Invention

### Problem to be Solved by the Invention

In the monitoring system disclosed in Patent Document 1, the activation of the alarm device is prevented if two or more persons are present in the area where the monitored person is present. Therefore, it is conceivable that even though the situation is such that the alarm device should be activated, the activation of the alarm device is prevented.

For example, in a case where the monitored person is a resident of a welfare facility for the elderly, if, as a result of the resident's predetermined action (e.g., getting up, falling down, etc.), the resident is in need of assistance but no attendant who assists the resident is present in the area where the resident is present, the alarm device should be activated. However, in this case, if another resident is present in the area where that resident is present, the activation of the alarm device will be prevented.

An object of the present disclosure is to provide a monitoring system, a monitoring device, a monitoring method, and a monitoring program that easily ensure that an alarm is issued in situations where the issuance of an alarm is needed, while avoiding an alarm from being issued in situations where the issuance of an alarm is not needed.

### Means for Solving Problem

A monitoring system according to the present disclosure includes: an alarm device; a state detection unit configured to detect a state of a monitored person; an alarm control unit capable of activating the alarm device in response to a predetermined state being detected by the state detection unit; and a determination unit configured to determine whether or not a predetermined attribute person, who is different from the monitored person and has a predetermined attribute, is present in an area where the monitored person is present, wherein the alarm control unit does not activate the alarm device in response to (i) the predetermined state being detected by the state detection unit and (ii) the determination unit determining that the predetermined attribute person is present in the area, and the alarm control unit activates the alarm device in response to (i) the predetermined state being detected by the state detection unit and (ii) the determination unit determining that the predetermined attribute person is not present in the area.

According to the present disclosure, a monitoring method that is performed by a monitoring system includes: a state detection step of detecting a state of a monitored person; a determination step of determining whether or not a predetermined attribute person, who is different from the monitored person and has a predetermined attribute, is present in an area where the monitored person is present; and an alarm step of not activating an alarm device in response to (i) a predetermined state being detected in the state detection step and (ii) it being determined in the determination step that the predetermined attribute person is present in the area, and of activating the alarm device in response to (i) the predetermined state being detected in the state detection step and (ii) it being determined in the determination step that the predetermined attribute person is not present in the area.

A monitoring program according to the present disclosure causes a computer to realize: a state acquisition function of acquiring information indicating a state of a monitored person; a determination function of determining whether or not a predetermined attribute person, who is different from the monitored person and has a predetermined attribute, is present in an area where the monitored person is present; and an alarm function of not activating an alarm device in response to (i) information indicating a predetermined state being acquired by the state acquisition function and (ii) it being determined by the determination function that the predetermined attribute person is present in the area, and of activating the alarm device in response to (i) the predetermined state being detected by the state acquisition function and (ii) it being determined by the determination function that the predetermined attribute person is not present in the area.

According to the present disclosure, in response to the monitored person being in a predetermined state, the alarm device is not activated if the predetermined attribute person is present in the area where the monitored person is present, whereas the alarm device is activated if the predetermined attribute person is not present in the area.

With this, for example, in a case where the monitored person is a resident of a welfare facility for the elderly and the predetermined attribute is "being an attendant who assists the resident", if the resident who is the monitored person is in a predetermined state (e.g., state where he or she has fallen) and the attendant is present in the area where the resident is present, the alarm device is not activated. Also, if the resident who is the monitored person is in the predetermined state (e.g., state where the monitored person has fallen) and the attendant is not present in the area where the resident is present, the alarm device is activated regardless of the presence of another resident. That is, it is possible to avoid a situation where the presence of another resident prevents the issuance of an alarm.

Therefore, the present disclosure easily ensures that an alarm is issued in situations where the issuance of an alarm is needed, while avoiding an alarm from being issued in situations where the issuance of an alarm is not needed.

A monitoring device according to the present disclosure includes: a state detection unit configured to detect a state of a monitored person; a transmission unit capable of transmitting state information defined as information indicating a result of detection by the state detection unit, to the outside, in response to a predetermined state being detected by the state detection unit; and a determination unit configured to determine whether or not a predetermined attribute person, who is different from the monitored person and has a predetermined attribute, is present in an area where the monitored person is present, wherein the transmission unit does not transmit the state information to the outside in response to (i) the predetermined state being detected by the state detection unit and (ii) the determination unit determining that the predetermined attribute person is present in the area, and the transmission unit transmits the state information to the outside in response to (i) the predetermined state being detected by the state detection unit and (ii) the determination unit determining that the predetermined attribute person is not present in the area.

According to the present disclosure, in response to the monitored person entering a predetermined state, the state information is not transmitted to the outside if the predetermined attribute person is present in the area where the monitored person is present, and the state information is transmitted to the outside if the predetermined attribute person is not present in the area. That is, for example, in a configuration in which the state information is transmitted to an alarm device in the outside, the alarm device is not activated if the monitored person is in a predetermined state and the predetermined attribute person is present in the area where the monitored person is present, and the alarm device is activated if the monitored person is in a predetermined state and the predetermined attribute person is not present in the area.

With this, for example, in a case where the monitored person is a resident of a welfare facility for the elderly and the predetermined attribute is "being an attendant who assists the resident", if the resident who is the monitored person is in a predetermined state (e.g., state where he or she has fallen) and the attendant is present in the area where the resident is present, the alarm device is not activated. Also, if the resident who is the monitored person is in the predetermined state (e.g., state where he or she has fallen) and the attendant is not present in the area where the resident is present, the alarm device is activated regardless of the presence of another resident. That is, it is possible to avoid a situation where the presence of another resident prevents the issuance of an alarm.

Therefore, the present disclosure easily ensures that an alarm is issued in situations where the issuance of an alarm is needed, while avoiding an alarm from being issued in situations where the issuance of an alarm is not needed.

Furthermore, in the present disclosure, preferably, the predetermined state is a state where the monitored person is in danger.

According to this configuration, if the monitored person is in danger (for example, the monitored person has fallen) and the predetermined attribute person is not present in the area where the monitored person is present, the alarm device will be activated. With this, for example, in a case where the monitored person is a resident of a welfare facility for the elderly and the predetermined attribute is "being an attendant who assists the resident", the attendant can seek to assist the resident who is in danger in response to the issuance of an alarm.

Furthermore, in the present disclosure, preferably, the predetermined attribute is being an attendant who assists the monitored person.

According to this configuration, if the monitored person is in a predetermined state (for example, state where the monitored person has fallen) and the attendant is present in the area where the monitored person is present, the alarm device will not be activated. Also, if the monitored person is in a predetermined state (e.g., state where the monitored person has fallen) and the attendant is not present in the area where the monitored person is present, the alarm device is activated regardless of the presence of a person other than the monitored person. That is, it is possible to avoid a situation where the presence of a person other than the monitored person prevents the issuance of an alarm.

Therefore, according to this configuration, an alarm is issued in situations where the issuance of an alarm is needed, while avoiding an alarm from being issued in situations where the issuance of an alarm is not needed, enabling the attendant to seek to assist the monitored person in response to the issuance of an alarm.

Moreover, in the present disclosure, preferably, the monitoring system further includes a detection unit configured to detect a specific object worn by the attendant, and the determination unit determines whether or not the predetermined attribute person is present in the area based on a result of detection by the detection unit.

According to this configuration, whether or not the predetermined attribute person is present in the area where the monitored person is present is determined based on the detection of a specific object (such as e.g., an IC card or staff uniform). This makes it easier to simplify the determination algorithm compared to a configuration in which the presence or absence of predetermined attribute person is determined based on face identification, for example.

Moreover, in the present disclosure, preferably, the monitoring system further includes a biometric information acquisition unit configured to acquire at least one of biometric information of a person entering the area and biometric information of a person present in the area, and the determination unit determines whether or not the predetermined attribute person is present in the area based on the biometric information acquired by the biometric information acquisition unit.

According to this configuration, the presence or absence of the predetermined attribute person can be determined without relying on an object (such as, e.g., an IC card or staff uniform) worn by the predetermined attribute person. In other words, in order to determine whether or not a predetermined attribute person is present, it is not necessary for predetermined attribute persons to wear a common specific object. Therefore, there is no need to oblige the predetermined attribute persons to wear a specific object in order to operate the monitoring system. It is thus easy to facilitate the operation of the monitoring system.

Furthermore, in the present disclosure, preferably, the alarm control unit activates the alarm device in response to (i) the predetermined state being detected by the state detection unit, (ii) the determination unit determining that the predetermined attribute person is present in the area, and (iii) a predetermined first condition being satisfied.

Even in a case where the monitored person is in the predetermined state (for example, a state in which the monitored person has fallen), and the predetermined attribute person is present in the area where the monitored person is present, a case is conceivable in which an alarm should be issued depending on the situation. For example, if one predetermined attribute person is present in the area where the monitored person is present and the monitored person is in an abnormal state (e.g., a state of bleeding massively) such that the monitored person cannot be treated by the single predetermined attribute person, an alarm should be issued.

According to this configuration, in a situation where the alarm should be issued even when a predetermined attribute person is present in the area where the monitored person is present, the alarm device can be activated by setting the first condition as appropriate. This allows the user of the monitoring system (e.g., an attendant who assists the monitored person) to take appropriate action upon receiving the alarm.

Furthermore, in the present disclosure, preferably, the first condition is a condition that an abnormal state different from the predetermined state is detected by the state detection unit.

According to this configuration, even in the case where the monitored person is in the predetermined state (for example, state where the monitored person has fallen) and the predetermined attribute person is present in the area where the monitored person is present, an alarm will be issued if the monitored person is in an abnormal state (e.g., a state of bleeding massively). This allows the user of the monitoring system (e.g., an attendant who assists the monitored person) to take appropriate action upon receiving the alarm.

Moreover, in the present disclosure, preferably, the monitoring system further includes a person's state detection unit configured to detect a state of the predetermined attribute person, and the first condition is a condition that an abnormal state is detected by the person's state detection unit.

According to this configuration, even in the case where the monitored person is in the predetermined state (for example, state where the monitored person has fallen) and the predetermined attribute person is present in the area where the monitored person is present, an alarm will be issued if the predetermined attribute person is in an abnormal state (e.g., state of assaulting the monitored person). This allows the user of the monitoring system (e.g., an attendant who assists the monitored person) to take appropriate action upon receiving the alarm.

Furthermore, in the present disclosure, preferably, the first condition is a condition that the predetermined state continues for a predetermined period of time.

With this configuration, even in the case where the monitored person is in a predetermined state (e.g., a state that the person has fallen) and the predetermined attribute person is present in the area where the monitored person is present, an alarm is issued if the monitored person is in a predetermined state for a relatively long period of time. This allows the user of the monitoring system (e.g., an attendant who assists the monitored person) to take appropriate action upon receiving the alarm.

Furthermore, in the present disclosure, preferably, the alarm control unit does not activate the alarm device in response to (i) the predetermined state being detected by the state detection unit, (ii) the determination unit determining that the predetermined attribute person is not present in the area, and (iii) a predetermined second condition being satisfied.

Even in a case where the monitored person is in the predetermined state (for example, state where the monitored person has fallen), and no predetermined attribute person is present in the area where the monitored person is present, a case is conceivable in which an alarm should not be issued depending on the situation. For example, if the monitored person or the user of the monitoring system desires that an alarm is not issued during certain hours of the day, the alarm should not be issued during these hours of the day.

According to this configuration, in a situation where the alarm should not be issued even when a predetermined attribute person is not present in the area where the monitored person is present, the activation of the alarm device can be prevented by setting the second condition as appropriate. With this, it is possible to avoid situations where an alarm is issued when the alarm should not be issued.

Furthermore, in the present disclosure, preferably, the second condition is a condition that current time is included in preset hours of a day.

This can realize a configuration in which if the monitored person or the user of the monitoring system desires that an alarm is not issued during certain hours of the day, the alarm is not issued during these hours of the day.

Furthermore, in the present disclosure, preferably, the area is a room where the monitored person is present.

When the monitored person is in a predetermined state (for example, state where the monitored person has fallen) and a predetermined attribute person is present in a room where the monitored person is present, the issuance of an alarm is not needed because the predetermined attribute person can handle the monitored person in the predetermined state.

Also, when the monitored person is in a predetermined state and a predetermined attribute person is not present in the room where the monitored person is present, an alarm should be issued.

According to this configuration, in response to the monitored person being in a predetermined state, the alarm device is not activated if a predetermined attribute person is present in the room where the monitored person is present, whereas the alarm device is activated if the predetermined attribute person is not present in the room.

Therefore, according to this configuration, it is easy to ensure that an alarm is issued in situations where the issuance of an alarm is needed, while avoiding an alarm from being issued in situations where the issuance of an alarm is not needed.

Furthermore, in the present disclosure, preferably, the alarm device is a display device capable of issuing an alarm through image display.

According to this configuration, by displaying, for example, an image of the monitored person when issuing an alarm, the alarm device can realize a configuration in which the user of the monitoring system can understand the state of the monitored person in detail. With this, it is easier for the user of the monitoring system to take appropriate measures.

Furthermore, in the present disclosure, preferably, the alarm device is a call device capable of calling the predetermined attribute person.

According to this configuration, if the monitored person is in a predetermined state and the predetermined attribute person is not present in the area where the monitored person is present, the predetermined attribute person is called. With this, it is possible for the predetermined attribute person to take appropriate measures in response to being called.

### Brief Description of the Drawings

FIG. 1 is a diagram showing an example of a case where no predetermined attribute person is present in an area where a monitored person is present.
FIG. 2 is a diagram showing an example of a case where a predetermined attribute person is present in the area where the monitored person is present.
FIG. 3 is a block diagram of a monitoring system.
FIG. 4 is a flowchart of a flow of deciding whether to issue an alarm.
FIG. 5 is a diagram showing a monitoring method according to the present disclosure.

### Best Mode For Carrying Out The Invention

Embodiments for implementing the present disclosure will be described with reference to the drawings.

### [Overall Configuration of Monitoring System]

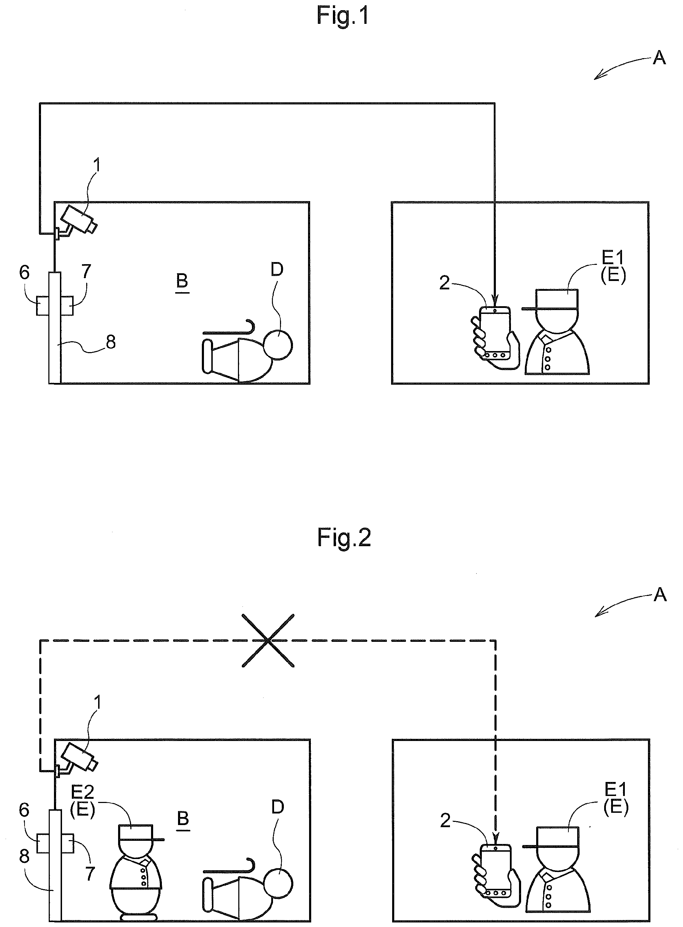

As shown in FIG. 1, a monitoring system A of the present embodiment includes a monitoring device 1 and an alarm device 2. The monitoring device 1 and the alarm device 2 are connected to each other via a given network.

The monitoring device 1 is installed in an area B where a monitored person D is present. The monitoring device 1 monitors the monitored person D. The monitoring device 1 can activate the alarm device 2 according to the state of the monitored person D. A predetermined attribute person E who possesses the alarm device 2 can understand the state of the monitored person D due to the alarm issued by the alarm device 2, while being outside the area B.

For example, in the example shown in FIG. 1, the monitored person D has fallen. In response thereto, the monitoring device 1 can activate the alarm device 2.

Note that in the present embodiment, the monitored person D is a resident of a welfare facility for the elderly, and the area B is a room of the welfare facility for the elderly where the monitored person D resides. That is, the area B in the present embodiment is the room where the monitored person D is present. Note that the present disclosure is not limited to this configuration, and the area B does not need to be a room. For example, if the monitored person D is present in a hallway, the area B is this hallway. Also, if the monitored person D is present in a relatively large space (such as, e.g., an outdoor plaza), the area B may be the entire space or may be only the vicinity of the monitored person D in this space. The area B is preferably within a range where another person (predetermined attribute person E) can visually check the state of the monitored person D.

Also, the predetermined attribute person E is a person who is different from the monitored person D and has a predetermined attribute. In the present embodiment, the predetermined attribute is being an attendant who assists the monitored person D. That is, the predetermined attribute person E is an attendant (staff) in the welfare facility for the elderly.

Note however that the present disclosure is not limited to this configuration. "A predetermined attribute" may be an attribute other than the attribute of "being an attendant who assists the monitored person D". For example, "a predetermined attribute" may be "being a relative of the monitored person D or an attendant who assists the monitored person D", or may be being a specific person (for example "a specific X or Y").

Note that in the present embodiment, the predetermined attribute person E is also a user of the monitoring system A.

The alarm device 2 of the present embodiment is an information terminal (such as a smartphone or tablet) held by the attendant. Also, in the example shown in FIG. 1, the predetermined attribute person E, who is an attendant, is located in a room (e.g., anteroom) other than the room where the monitored person D is located.

The monitoring system A may include one monitoring device 1 or a plurality of monitoring devices 1. That is, the monitoring system A may be a system for monitoring one monitored person D or a system for monitoring a plurality of monitored persons D.

A plurality of monitoring devices 1 may be installed in the welfare facility for the elderly. For example, a monitoring device 1 may be installed in each room of the welfare facility for the elderly, or may be installed in a region (such as, e.g., hallway) other than the rooms.

A single predetermined attribute person E or a plurality of predetermined attribute persons E may be present in the welfare facility for the elderly. The monitoring system A may include one alarm device 2 or a plurality of alarm devices 2. A plurality of predetermined attribute persons E may each hold one alarm device 2. The alarm device 2 may be a personal computer, a display, a lamp, a speaker, or the like installed in a room (e.g., anteroom) for the predetermined attribute person E. The alarm device 2 may be held by a person (e.g., a relative of the monitored person D) who is not the predetermined attribute person E, or may be a personal computer, a display, a lamp, a speaker, or the like installed in the home of a person (e.g., a relative of the monitored person D) who is not the predetermined attribute person E.

In the example shown in FIG. 2, a first person E1 and a second person E2 are shown. The first person E1 and the second person E2 are both predetermined attribute persons E.

The first person E1 is located in a room (e.g., anteroom) different from the area B, which is a room where the monitored person D is located. The second person E2 is located in the area B, which is a room where the monitored person D is located. Also, in the example shown in FIG. 2, the monitored person D has fallen as in the example shown in FIG. 1. In this case, in the monitoring system A of the present embodiment, the monitoring device 1 does not essentially activate the alarm device 2.

Hereinafter, the monitoring system A will be described in detail.

### [State Detection Unit]

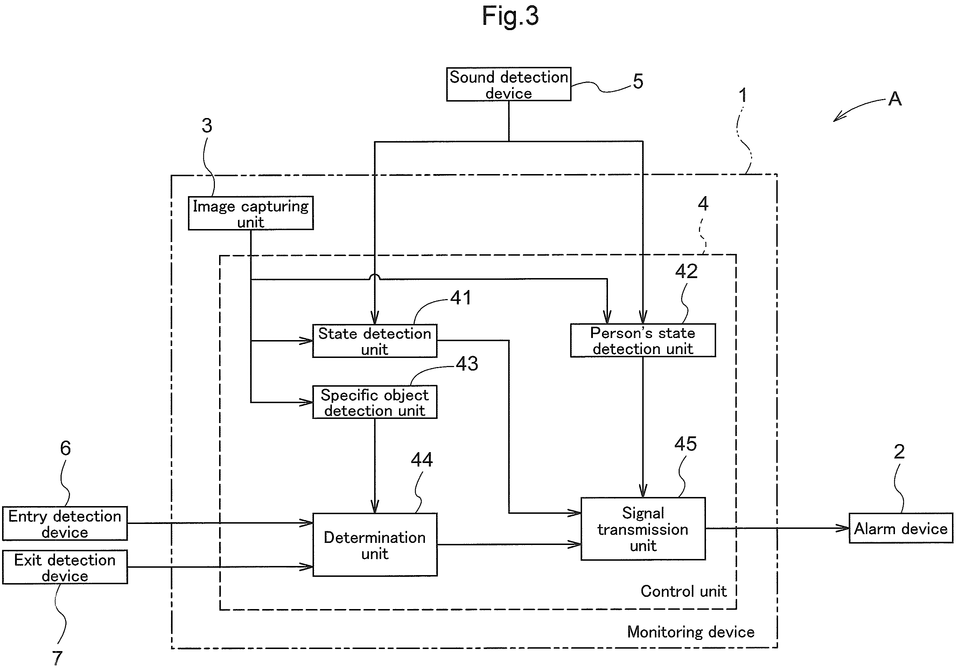

As shown in FIG. 3, the monitoring device 1 includes an image capturing unit 3 and a control unit 4. The image capturing unit 3 captures an image of the inside of the area B (see FIG. 1). Note that the image capture range of the image capturing unit 3 may be the entirety of the area B or may be part of the area B. Also, the image capture range of the image capturing unit 3 may include the outside of the area B.

In the present embodiment, the image capturing unit 3 is constituted by an infrared camera. Note however that the present disclosure is not limited to this configuration, and the image capturing unit 3 may be any device other than an infrared camera as long as it can capture an image of the inside of the area B. The image capturing unit 3 is preferably a ToF (Time of Flight) camera. A captured image obtained by the image capturing unit 3 is preferably a depth image, which is an image including depth information. Note that the captured image obtained by the image capturing unit 3 may be a moving image or a still image.

As shown in FIG. 3, the control unit 4 includes a state detection unit 41. The image capturing unit 3 transmits an obtained captured image to the state detection unit 41.

Also, as shown in FIG. 3, the monitoring system A includes a sound detection device 5 for detecting sound. The sound detection device 5 may be installed in the area B. The sound detection device 5 may be a known microphone. The sound detection device 5 transmits information indicating detected sound to the state detection unit 41.

The state detection unit 41 detects the state of the monitored person D based on the captured image received from the image capturing unit 3 and the information indicating the sound received from the sound detection device 5. That is, the monitoring system A includes the state detection unit 41 that detects the state of the monitored person D.

In the present embodiment, the state detection unit 41 generates a skeleton model that is information indicating the skeleton of the monitored person D, based on the received captured image. The skeletal model is generated, by analyzing the captured image to three-dimensionally recognize the positions of joints of the monitored person D and connecting the joints with straight lines.

Furthermore, the state detection unit 41 is configured to detect the body position (e.g., standing, lying, or sitting) of the monitored person D based on the captured image and the generated skeleton model, and to detect whether or not the monitored person D is in a predetermined state and whether or not the monitored person D is in an abnormal state different from the predetermined state, based on the captured image, the skeleton model, and the information indicating sound.

Although not particularly limited, the predetermined state may be a dangerous state (e.g., a fallen state) of the monitored person D. Also, although not particularly limited, the abnormal state may be a state in which the monitored person D is massively bleeding or a state in which the monitored person D is being assaulted by the predetermined attribute person E. Note that techniques for detecting a dangerous state such as a fallen state or an abnormal state based on a captured image or a skeleton model are known and will not be described here.

Also, the state detection unit 41 may be configured to determine whether or not the monitored person D is screaming based on information indicating sound, and to detect a dangerous state or an abnormal state of the monitored person D based on the result of this determination.

Note that there may be a case where a person other than the monitored person D is present in the image capture range of the image capturing unit 3. Therefore, the state detection unit 41 is preferably capable of recognizing a person in a captured image. By recognizing a person in the captured image, it is possible to avoid the situation where the state detection unit 41 mistakenly detects the state of a person other than the monitored person D. This can avoid the situation where, for example, when a person (e.g., predetermined attribute person E) other than the monitored person D is in a predetermined state (e.g., a fallen state), the state detection unit 41 mistakenly detects that the monitored person D is in a predetermined state.

Note that the method for recognizing a person in a captured image by the state detection unit 41 is not particularly limited, and a machine-learned neural network may be used to analyze the captured image and recognize the person.

### [Person's State Detection Unit]

As shown in FIG. 3, the control unit 4 includes a person's state detection unit 42. The image capturing unit 3 transmits an obtained captured image to the person's state detection unit 42. Also, the sound detection device 5 transmits information indicating detected sound to the person's state detection unit 42.

The person's state detection unit 42 detects the state of the predetermined attribute person E (see FIG. 2) present in the area B, based on the captured image received from the image capturing unit 3 and the information indicating sound received from the sound detection device 5. That is, the monitoring system A further includes the person's state detection unit 42 that detects the state of the predetermined attribute person E.

In the present embodiment, the person's state detection unit 42 generates a skeleton model that is information indicating the skeleton of the predetermined attribute person E, based on the received captured image. The skeleton model is generated, by analyzing the captured image to three-dimensionally recognize the positions of joints of the predetermined attribute person E and connecting the joints with straight lines.

Furthermore, the person's state detection unit 42 is configured to detect the body position (e.g., standing, lying, or sitting) of the predetermined attribute person E based on the captured image and the generated skeleton model, and to detect whether or not the predetermined attribute person E is in an abnormal state based on the captured image, the skeleton model, and the information indicating sound.

Although not particularly limited, the abnormal state may be a state where the predetermined attribute person E is assaulting the monitored person D. For example, if the predetermined attribute person E continues to move violently for a predetermined period of time or if the predetermined attribute person E makes a predetermined number of movements to wave his or her hands down, the predetermined attribute person E may be detected as being in an abnormal state (state of assaulting the monitored person D).

Also, the person's state detection unit 42 may be configured to determine whether or not the predetermined attribute person E is shouting in anger based on information indicating sound, and to detect that the predetermined attribute person E is in an abnormal state based on the result of this determination.

Note that the person's state detection unit 42 is preferably capable of recognizing a person in a captured image. By recognizing a person in the captured image, it is possible to avoid the situation where the person's state detection unit 42 mistakenly detects the state of a person other than the predetermined attribute person E. This can avoid the situation where, for example, when a person (e.g., monitored person D) other than the predetermined attribute person E is in an abnormal state, the person's state detection unit 42 mistakenly detects that the predetermined attribute person E is in an abnormal state.

Note that the method for recognizing a person in a captured image by the person's state detection unit 42 is not particularly limited, and a machine-learned neural network may be used to analyze the captured image and recognize the person.

### [Determination Unit]

As shown in FIG. 3, the control unit 4 includes a specific object detection unit 43 (corresponding to a "detection unit" according to the present disclosure). The image capturing unit 3 transmits an obtained captured image to the specific object detection unit 43. The specific object detection unit 43 performs image analysis based on the captured image received from the image capturing unit 3 to detect a specific object. A specific object refers to an object worn by the predetermined attribute person E who is an attendant in the welfare facility for the elderly. That is, the monitoring system A includes the specific object detection unit 43 that detects a specific object worn by the attendant.

Note that the specific object is not particularly limited and may be an attendant's uniform or badge, or the like, for example.

If the predetermined attribute person E is present in the area B, the specific object will appear in the captured image and will be detected by the specific object detection unit 43. If the predetermined attribute person E is not present in the area B, the specific object does not appear in the captured image and thus is not detected by the specific object detection unit 43.

Also, as shown in FIGS. 1 to 3, the monitoring system A includes an entry detection device 6 (corresponding to a "biometric information acquisition unit" according to the present disclosure) and an exit detection device 7. The entry detection device 6 and the exit detection device 7 are both installed in the vicinity of a door 8 provided in a doorway of the area B.

The entry detection device 6 is configured to acquire biometric information of a person entering the area B. That is, the monitoring system A includes the entry detection device 6 that acquires biometric information of a person entering the area B. For example, a configuration is possible in which the door 8 is normally closed, and in response to the entry detection device 6 acquiring biometric information, the door 8 is temporarily opened. Note that the biometric information acquired by the entry detection device 6 is not particularly limited and may be face information or fingerprint information, for example.

The exit detection device 7 is configured to acquire biometric information of a person exiting the area B. For example, a configuration is possible in which the door 8 is normally closed, and in response to the exit detection device 7 acquiring biometric information, the door 8 is temporarily opened. Note that the biometric information acquired by the exit detection device 7 is not particularly limited and may be face information or fingerprint information, for example.

As shown in FIG. 3, the control unit 4 includes a determination unit 44. The result of the detection by the specific object detection unit 43 is transmitted to the determination unit 44. Also, the pieces of biometric information acquired by the entry detection device 6 and the exit detection device 7 are transmitted to the determination unit 44.

The determination unit 44 determines whether or not the predetermined attribute person E is present in the area B, based on the result of the detection by the specific object detection unit 43 and the pieces of biometric information acquired by the entry detection device 6 and the exit detection device 7.

That is, the monitoring system A includes the determination unit 44 that determines whether or not the predetermined attribute person E, who is different from the monitored person D and has a predetermined attribute, is present in the area B where the monitored person D is present. Also, the determination unit 44 determines whether or not the predetermined attribute person E is present in the area B based on the result of the detection by the specific object detection unit 43. The determination unit 44 also determines whether or not the predetermined attribute person E is present in the area B based on the biometric information acquired by the entry detection device 6.

The following will describe in detail the determination performed based on the biometric information acquired by the entry detection device 6 and the exit detection device 7. The determination unit 44 has stored therein biometric information (e.g., face information or fingerprint information) of all attendants in this welfare facility for the elderly. The determination unit 44 determines whether or not the person who has entered the area B is the predetermined attribute person E, by comparing the biometric information stored in the determination unit 44 with the biometric information acquired by the entry detection device 6. The determination unit 44 also determines whether or not the person who has exited the area B is the predetermined attribute person E, by comparing the biometric information stored in the determination unit 44 with the biometric information acquired by the exit detection device 7.

Also, the determination unit 44 determines whether the history of the biometric information acquired by the entry detection device 6 and the exit detection device 7 has a first pattern, a second pattern, or a third pattern. The first pattern is a pattern in which the predetermined attribute person E has entered the area B and then the predetermined attribute person E has not exited the area B. The second pattern is a pattern in which the predetermined attribute person E has entered the area B and then the predetermined attribute person E has exited the area B. The third pattern is a pattern in which the predetermined attribute person E has not entered the area B.

If a specific object is detected by the specific object detection unit 43, the determination unit 44 determines that the predetermined attribute person E is present in the area B regardless of the biometric information acquired by the entry detection device 6 and the exit detection device 7.

Also, if the history of the biometric information acquired by the entry detection device 6 and the exit detection device 7 has the above-described first pattern, the determination unit 44 determines that the predetermined attribute person E is present in the area B regardless of the result of the detection by the specific object detection unit 43.

Also, if no specific object is detected by the specific object detection unit 43 and the history of the biometric information acquired by the entry detection device 6 and the exit detection device 7 has the above-described second or third pattern, the determination unit 44 determines that the predetermined attribute person E is not present in the area B.

Note that the present disclosure is not limited to this configuration. A configuration is also possible in which, only if a specific object is detected by the specific object detection unit 43 and the history of the biometric information acquired by the entry detection device 6 and the exit detection device 7 has the first pattern, the determination unit 44 determines that the predetermined attribute person E is present in the area B.

### [Signal Transmission Unit]

As shown in FIG. 3, the control unit 4 includes a signal transmission unit 45 (corresponding to an "alarm control unit" or a "transmission unit" according to the present disclosure). The results of the detection by the state detection unit 41 and the person's state detection unit 42 are transmitted to the signal transmission unit 45. Also, the result of the determination by the determination unit 44 is transmitted to the signal transmission unit 45.

The signal transmission unit 45 is configured to be able to transmit a predetermined alarm signal (corresponding to "state information" according to the present disclosure) to the alarm device 2 in response to a predetermined state being detected by the state detection unit 41. The alarm signal is information indicating a result of detection by the state detection unit 41.

The alarm device 2 is activated upon receiving the alarm signal. More specifically, the alarm device 2 issues an alarm by image display and a sound that calls the predetermined attribute person E. In other words, the alarm device 2 is a display device capable of issuing an alarm through image display and is a call device capable of calling the predetermined attribute person E.

Note that the image display may be display of a captured image captured by the image capturing unit 3. Also, content of the image display and content of the calling sound may inform the name of the monitored person D, the location of the room (such as the room number), the current state, and the like, for example. The alarm device 2 may also be capable of issuing an alarm through vibration.

That is, the monitoring system A includes the signal transmission unit 45 capable of activating the alarm device 2 in response to a predetermined state being detected by the state detection unit 41. Also, the monitoring device 1 includes the signal transmission unit 45 capable of transmitting an alarm signal that is information indicating the result of detection by the state detection unit 41, to the outside in response to a predetermined state being detected by the state detection unit 41.

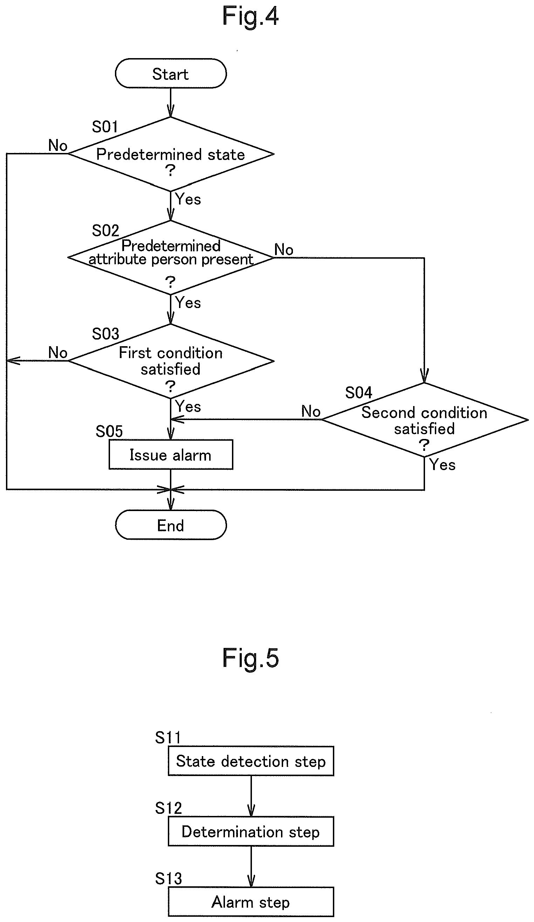

The signal transmission unit 45 is configured to decide whether or not to transmit the above-described alarm signal to the alarm device 2 in accordance with an alarm decision flow shown in FIG. 4, based on results of the detection by the state detection unit 41 and the person's state detection unit 42, and a result of the determination by the determination unit 44.

### [Alarm Decision Flow]

Upon start of the alarm decision flow shown in FIG. 4, processing in step S01 is first executed. In step S01, it is determined whether or not a predetermined state has been detected by the state detection unit 41. If it is determined that the predetermined state has not been detected by the state detection unit 41 ("No" in step S01 in FIG. 4), the alarm decision flow is temporarily ended. If it is determined that the predetermined state has been detected by the state detection unit 41 ("Yes" in step S01 in FIG. 4), the processing moves to step S02.

In step S02, it is determined whether or not the determination unit 44 has determined that the predetermined attribute person E is present in the area B. If it is determined that the predetermined attribute person E is present in the area B ("Yes" in step S02 in FIG. 4), the processing moves to step S03. Also, if it is determined that the predetermined attribute person E is not present in the area B ("No" in step S02 in FIG. 4), the processing moves to step S04.

In step S03, it is determined whether or not a predetermined first condition is satisfied. The first condition of the present embodiment is the condition that an abnormal state has been detected by the person's state detection unit 42. Note however that the present disclosure is not limited to this configuration. The first condition may be, for example, the condition that an abnormal state different from the predetermined state has been detected by the state detection unit 41 or the condition that the predetermined state continues for a predetermined period of time (e.g., for five minutes).

If the first condition is satisfied ("Yes" in step S03 in FIG. 4), the processing moves to step S05.

In step S05, the signal transmission unit 45 transmits the above-described alarm signal to the alarm device 2. That is, in this case, the signal transmission unit 45 activates the alarm device 2. Then, this alarm decision flow is temporarily ended.

In this way, in a case where the predetermined state is detected by the state detection unit 41 and it is determined by the determination unit 44 that the predetermined attribute person E is present in the area B, if the predetermined first condition is satisfied, the signal transmission unit 45 activates the alarm device 2.

If the first condition is not satisfied ("No" in step S03 in FIG. 4), the alarm decision flow is temporarily ended. Accordingly, in this case, the signal transmission unit 45 does not transmit the above-described alarm signal to the alarm device 2. That is, in this case, the signal transmission unit 45 does not activate the alarm device 2.

In step S04, it is determined whether or not a predetermined second condition is satisfied. The second condition of the present embodiment is the condition that the current time is included in preset hours of the day. The hours of the day may be time set by being input in advance according to the will of the monitored person D or an agreement on the monitoring of the monitored person D (e.g., an agreement on the monitoring time), for example, or may be time for care of the monitored person D, such as time for which the predetermined attribute person E wipes the body of the monitored person D or drain time.

Note however that the present disclosure is not limited to this configuration. The second condition may be, for example, the condition that the time at which the predetermined state is detected by the state detection unit 41 is included in preset hours of the day.

If the second condition is satisfied ("Yes" in step S04 in FIG. 4), the alarm decision flow is temporarily ended. Accordingly, in this case, the signal transmission unit 45 does not transmit the above-described alarm signal to the alarm device 2. That is, in this case, the signal transmission unit 45 does not activate the alarm device 2.

In this way, in a case where the predetermined state is detected by the state detection unit 41 and it is determined by the determination unit 44 that the predetermined attribute person E is not present in the area B, if the predetermined second condition is satisfied, the signal transmission unit 45 does not activate the alarm device 2.

If the second condition is not satisfied ("No" in step S04 in FIG. 4), the processing moves to step S05. In this case, the signal transmission unit 45 transmits the above-described alarm signal to the alarm device 2. That is, in this case, the signal transmission unit 45 activates the alarm device 2. Then, this alarm decision flow is temporarily ended.

As is clear from the description above, in a case where the predetermined state is detected by the state detection unit 41 ("Yes" in step S01 in FIG. 4) and it is determined by the determination unit 44 that the predetermined attribute person E is not present in the area B ("No" in step S02 in FIG. 4), the signal transmission unit 45 transmits an alarm signal except if the second condition is satisfied. That is, if the predetermined state is detected by the state detection unit 41 and it is determined by the determination unit 44 that the predetermined attribute person E is not present in the area B, the signal transmission unit 45 activates the alarm device 2. Also, if the predetermined state is detected by the state detection unit 41 and it is determined by the determination unit 44 that the predetermined attribute person E is not present in the area B, the signal transmission unit 45 transmits an alarm signal to the outside.

With this configuration, as shown in FIG. 1, if the monitored person D is in a predetermined state (e.g., state where the monitored person D has fallen) and the predetermined attribute person E is not present in the area B where the monitored person D is present, the monitoring device 1 transmits an alarm signal to the alarm device 2. As a result, the monitoring device 1 activates the alarm device 2.

Also, as is clear from the description above, if the predetermined state is detected by the state detection unit 41 ("Yes" in step S01 in FIG. 4) and it is determined by the determination unit 44 that the predetermined attribute person E is present in the area B ("Yes" in step S02 in FIG. 4), the signal transmission unit 45 does not transmit any alarm signal except if the first condition is satisfied. That is, if the predetermined state is detected by the state detection unit 41 and it is determined by the determination unit 44 that the predetermined attribute person E is present in the area B, the signal transmission unit 45 does not activate the alarm device 2. Also, if the predetermined state is detected by the state detection unit 41 and it is determined by the determination unit 44 that the predetermined attribute person E is present in the area B, the signal transmission unit 45 does not transmit any alarm signal to the outside.

With this configuration, as shown in FIG. 2, if the monitored person D is in a predetermined state (e.g., state where the monitored person D has fallen) and the predetermined attribute person E is present in the area B where the monitored person D is present, no alarm signal is transmitted from the monitoring device 1 to the alarm device 2. In other words, the monitoring device 1 does not activate the alarm device 2.

Note that the functional units such as the control unit 4 and the state detection unit 41 included in the control unit 4 may be physical devices such as a microcomputer or may also be functional units of software. For example, a configuration is also possible in which programs corresponding to the functional units included in the control unit 4 are stored in a not-shown ROM or nonvolatile memory, and the processes corresponding to the functional units are executed by loading these programs into the CPU and executing the programs.

According to the above-described configurations, in response to the monitored person D being in a predetermined state, the alarm device 2 is not activated if a predetermined attribute person E is present in the area B where the monitored person D is present, whereas the alarm device 2 is activated if the predetermined attribute person E is not present in the area B.

With this, for example, in a case where the monitored person D is a resident of a welfare facility for the elderly and the predetermined attribute is "being an attendant who assists the resident", if the resident who is the monitored person D is in a predetermined state (e.g., state where he or she has fallen) and the attendant is present in the area B where the resident is present, the alarm device 2 is not activated. Also, if the resident who is the monitored person D is in the predetermined state (e.g., state where the monitored person D has fallen) and the attendant is not present in the area B where the resident is present, the alarm device 2 is activated regardless of the presence of another resident. That is, it is possible to avoid a situation where the presence of another resident prevents the issuance of an alarm.

Therefore, the above-described configuration easily ensures that an alarm is issued in situations where the issuance of an alarm is needed, while avoiding an alarm from being issued in situations where the issuance of an alarm is not needed.

### [Other Embodiments]

(1) The present invention may be configured as a monitoring method in which processing executed by the corresponding members in the above-described embodiment is executed by one or more steps. For example, as shown in FIG. 5, the present invention may be configured as a monitoring method including the state detection step S11 corresponding to the state detection unit 41, the determination step S12 corresponding to the determination unit 44, and the alarm step S13 corresponding to the signal transmission unit 45.
   In this case, the alarm step S13 is a step of not activating the alarm device 2 in response to (i) the predetermined state being detected in the state detection step S11 and (ii) it being determined in the determination step S12 that the predetermined attribute person E is present in the area B, and of activating the alarm device 2 in response to (i) the predetermined state being detected in the state detection step S11 and (ii) it being determined in the determination step S12 that the predetermined attribute person E is not present in the area B.
(2) The present invention may be configured as a monitoring program causing a computer to execute the functions of the members in the present embodiment. For example, the present invention may be configured as a monitoring program causing a computer to execute the state acquisition function corresponding to the state detection unit 41, the determination function corresponding to the determination unit 44, and the alarm function corresponding to the signal transmission unit 45.

In this case, the state acquisition function refers to a function of acquiring information indicating the state of the monitored person D. Specifically, "information indicating the state of the monitored person D" may be a captured image acquired by the image capturing unit 3 or may be information indicating the state of the monitored person D detected based on the captured image.

Also, in this case, the alarm function refers to a function of not activating the alarm device 2 in response to (i) information indicating the predetermined state being acquired by the state acquisition function and (ii) it being determined by the determination function that the predetermined attribute person E is present in the area B, and of activating the alarm device 2 in response to (i) the predetermined state being detected by the state acquisition function and (ii) it being determined by the determination function that the predetermined attribute person E is not present in the area B.

The monitoring program may be stored, for example, in a portable terminal such as a smartphone and may be configured to be executed in this portable terminal. In this case, the portable terminal may be configured to function as an "alarm device" according to the present disclosure.

(3) Some or all of the state detection unit 41, the person's state detection unit 42, the specific object detection unit 43, the determination unit 44, and the signal transmission unit 45 may be provided outside the monitoring device 1 (e.g., in a personal computer used by the predetermined attribute person E).

(4) In the alarm decision flow shown in FIG. 4, step S03 may be omitted or step S04 may be omitted.

(5) The sound detection device 5 may be included in the monitoring device 1.

(6) The entry detection device 6 and the exit detection device 7 may be configured as devices that read an IC card worn by an attendant. In this case, the IC card corresponds to a "specific object" according to the present disclosure, and both the entry detection device 6 and the exit detection device 7 correspond to a "detection unit" according to the present disclosure.

(7) The monitoring device 1 or the monitoring system A may include a facial information acquisition unit that acquires facial information of a person. In this case, the facial information corresponds to "biometric information" according to the present disclosure and the facial information acquisition unit corresponds to the "biometric information acquisition unit" according to the present disclosure. The facial information acquisition unit may be configured to acquire only one type of the facial information of a person entering the area B and the facial information of a person present in the area B, or may be configured to acquire both. That is, the facial information acquisition unit acquires at least one type of the facial information of a person entering the area B and the facial information of a person present in the area B. The facial information acquisition unit may acquire, for example, facial information of a person present in the area B based on a captured image acquired by the image capturing unit 3. Also, the determination unit 44 may be configured to determine whether or not the predetermined attribute person E is present in the area B, based on the facial information acquired by the facial information acquisition unit.

(8) The image capturing unit 3 may be omitted. In this case, the state detection unit 41 may detect the state of the monitored person D, for example, only based on the information indicating sound received from the sound detection device 5.

(9) The specific object detection unit 43 may be omitted. In this case, the determination unit 44 may determine whether or not the predetermined attribute person E is present in the area B, for example, only based on the biometric information acquired by the entry detection device 6 and the exit detection device 7.

(10) The entry detection device 6 and the exit detection device 7 may be omitted. In this case, the determination unit 44 may determine whether or not the predetermined attribute person E is present in the area B, for example, only based on the result of the detection by the specific object detection unit 43.

(11) An input unit may be provided that can be used by the user, upon activation of the alarm device 2, to input information indicating whether or not the issuance of an alarm was actually not needed. An information storage unit may further be provided that stores various information (such as e.g., information indicating the body position and location of the monitored person D, or the time) when the alarm device 2 is activated, upon input of information indicating that the issuance of an alarm was actually not needed to the input unit. Moreover, a configuration is also possible in which the signal transmission unit 45 determines, based on the information stored in the information storage unit, whether or not the current situation is similar to the situation at that time (when the alarm device 2 was activated despite the fact that issuance of an alarm was not needed) and, if it is determined that the situation is similar, the signal transmission unit 45 does not transmit the above-described alarm signal to the alarm device 2.

If the alarm device 2 is activated despite the situation where issuance of an alarm is actually not needed, this configuration will make it easier to avoid the situation where the alarm is issued every time a similar situation arises.

Note that the configurations disclosed in the above-described embodiments (including other embodiments: the same applies hereinafter) can be applied in combination with the configurations disclosed in other embodiments, as long as no contradiction arises. Also, the embodiments disclosed herein are examples, and the embodiments of the present disclosure are not limited thereto, and modifications are possible as appropriate within the scope of not departing from the purpose of the present disclosure.

### Industrial Applicability

The present disclosure can be used in not only welfare facilities for the elderly but also various facilities such as medical facilities and public facilities, and can also be used even outdoors.

## Claims

1. A monitoring system comprising:
an alarm device;
a state detection unit configured to detect a state of a monitored person;
an alarm control unit capable of activating the alarm device in response to a predetermined state being detected by the state detection unit; and
a determination unit configured to determine whether or not a predetermined attribute person, who is different from the monitored person and has a predetermined attribute, is present in an area where the monitored person is present,
wherein the alarm control unit does not activate the alarm device in response to (i) the predetermined state being detected by the state detection unit and (ii) the determination unit determining that the predetermined attribute person is present in the area, and
the alarm control unit activates the alarm device in response to (i) the predetermined state being detected by the state detection unit and (ii) the determination unit determining that the predetermined attribute person is not present in the area.

2. The monitoring system according to claim 1,
wherein the predetermined state is a state where the monitored person is in danger.

3. The monitoring system according to claim 1 or 2,
wherein the predetermined attribute is being an attendant who assists the monitored person.

4. The monitoring system according to claim 3, further comprising:
a detection unit configured to detect a specific object worn by the attendant,
wherein the determination unit determines whether or not the predetermined attribute person is present in the area based on a result of detection by the detection unit.

5. The monitoring system according to claim 1 or 2, further comprising:
a biometric information acquisition unit configured to acquire at least one of biometric information of a person entering the area and biometric information of a person present in the area,
wherein the determination unit determines whether or not the predetermined attribute person is present in the area based on the biometric information acquired by the biometric information acquisition unit.

6. The monitoring system according to claim 1 or 2,
wherein the alarm control unit activates the alarm device in response to (i) the predetermined state being detected by the state detection unit, (ii) the determination unit determining that the predetermined attribute person is present in the area, and (iii) a predetermined first condition being satisfied.

7. The monitoring system according to claim 6,
wherein the first condition is a condition that an abnormal state different from the predetermined state is detected by the state detection unit.

8. The monitoring system according to claim 6, further comprising:
a person's state detection unit configured to detect a state of the predetermined attribute person,
wherein the first condition is a condition that an abnormal state is detected by the person's state detection unit.

9. The monitoring system according to claim 6,
wherein the first condition is a condition that the predetermined state continues for a predetermined period of time.

10. The monitoring system according to claim 1 or 2,
wherein the alarm control unit does not activate the alarm device in response to (i) the predetermined state being detected by the state detection unit, (ii) the determination unit determining that the predetermined attribute person is not present in the area, and (iii) a predetermined second condition is satisfied.

11. The monitoring system according to claim 10,
wherein the second condition is a condition that current time is included in preset hours of a day.

12. The monitoring system according to claim 1 or 2,
wherein the area is a room where the monitored person is present.

13. The monitoring system according to claim 1 or 2,
wherein the alarm device is a display device capable of issuing an alarm through image display.

14. The monitoring system according to claim 1 or 2,
wherein the alarm device is a call device capable of calling the predetermined attribute person.

15. A monitoring device comprising:
a state detection unit configured to detect a state of a monitored person;
a transmission unit capable of transmitting state information defined as information indicating a result of detection by the state detection unit, to the outside, in response to a predetermined state being detected by the state detection unit; and
a determination unit configured to determine whether or not a predetermined attribute person, who is different from the monitored person and has a predetermined attribute, is present in an area where the monitored person is present,
wherein the transmission unit does not transmit the state information to the outside in response to (i) the predetermined state being detected by the state detection unit and (ii) the determination unit determining that the predetermined attribute person is present in the area, and
the transmission unit transmits the state information to the outside in response to (i) the predetermined state being detected by the state detection unit and (ii) the determination unit determining that the predetermined attribute person is not present in the area.

16. A monitoring method performed by a monitoring system, the method comprising:
a state detection step of detecting a state of a monitored person;
a determination step of determining whether or not a predetermined attribute person, who is different from the monitored person and has a predetermined attribute, is present in an area where the monitored person is present; and
an alarm step of not activating an alarm device in response to (i) a predetermined state being detected in the state detection step and (ii) it being determined in the determination step that the predetermined attribute person is present in the area, and of activating the alarm device in response to (i) the predetermined state being detected in the state detection step and (ii) it being determined in the determination step that the predetermined attribute person is not present in the area.

17. A monitoring program causing a computer to realize:
a state acquisition function of acquiring information indicating a state of a monitored person;
a determination function of determining whether or not a predetermined attribute person, who is different from the monitored person and has a predetermined attribute, is present in an area where the monitored person is present; and
an alarm function of not activating an alarm device in response to (i) information indicating a predetermined state being acquired by the state acquisition function and (ii) it being determined by the determination function that the predetermined attribute person is present in the area, and of activating the alarm device in response to (i) the predetermined state being detected by the state acquisition function and (ii) it being determined by the determination function that the predetermined attribute person is not present in the area.
